(19) Europäisches Patentamt
European Patent Office
Office européen des brevets

(11) Veröffentlichungsnummer: **0 346 683**
**A1**

(12) **EUROPÄISCHE PATENTANMELDUNG**

(21) Anmeldenummer: **89109808.9**

(51) Int. Cl.4: **C07D 207/327**

(22) Anmeldetag: **31.05.89**

Patentansprüche für folgende Vertragsstaaten: ES + GR

(30) Priorität: **14.06.88 DE 3820190**

(43) Veröffentlichungstag der Anmeldung: **20.12.89 Patentblatt 89/51**

(84) Benannte Vertragsstaaten: **AT BE CH DE ES FR GB GR IT LI NL SE**

(71) Anmelder: **CASSELLA Aktiengesellschaft**
**Hanauer Landstrasse 526**
**D-6000 Frankfurt am Main 61(DE)**

(72) Erfinder: **Zoller, Gerhard, Dr.**
**Höhenstrasse 8**
**D-6369 Schöneck(DE)**
Erfinder: **Beyerle, Rudi, Dr.**
**An der Pfaffenmauer 44**
**D-6000 Frankfurt am Main 60(DE)**
Erfinder: **Schindler, Ursula, Dr.**
**Reinhardswaldweg 1**
**D-6082 Mörfelden-Walldorf(DE)**

(74) Vertreter: **Urbach, Hans-Georg, Dr. et al**
**Hanauer Landstrasse 526**
**D-6000 Frankfurt am Main 61(DE)**

(54) **Pyrrolderivate, ihre Herstellung und ihre Verwendung.**

(57) Pyrrolderivate der allgemeinen Formel I

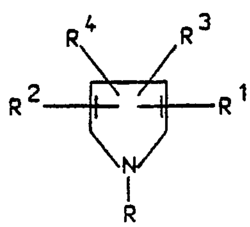

(I)

worin
R Alkyl, das durch $-NH_2$ oder Acylamino substituiert ist,
$R^1$, $R^2$ unabhängig voneinander Wasserstoff oder Alkyl mit 1 bis 4 Kohlenstoffatomen,
$R^3$ Wasserstoff, Alkyl mit 1 bis 4 Kohlenstoffatomen oder eine Carbonsäuregruppierung,
$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyclus mit 1 oder 2 Heteroatomen aus der Reihe N, O, S substituiert ist, bedeuten, sowie deren herstellbaren, pharmazeutisch akzeptablen Salze bzw. Säureadditionssalze sind wertvolle pharmakologische Wirkstoffe.

EP 0 346 683 A1

## Pyrrolderivate, ihre Herstellung und ihre Verwendung als pharmazeutische Wirkstoffe

Die vorliegende Erfindung betrifft Pyrrolderivate der allgemeinen Formel I

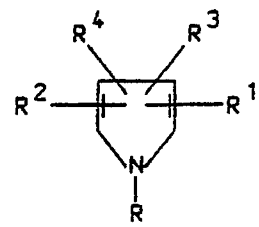

(I)

worin
R Alkyl, das durch $-NH_2$ oder Acylamino der allgemeinen Formel II

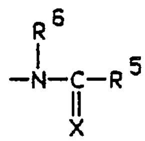

(II)

substituiert ist,
$R^1$, $R^2$ unabhängig voneinander Wasserstoff oder Alkyl mit 1 bis 4 Kohlenstoffatomen,
$R^3$ Wasserstoff, Alkyl mit 1 bis 4 Kohlenstoffatomen oder eine Carbonsäuregruppierung,
$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyclus mit 1 oder 2 Heteroatomen aus der Reihe N, O, S substituiert ist,
$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5 Kohlenstoffatomen oder gegebenenfalls substituiertes Phenylamino,
$R^6$ Wasserstoff oder Alkyl mit 1 bis 4 Kohlenstoffatomen, wobei $R^5$ und $R^6$ auch zusammen mit der

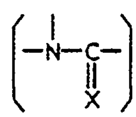 - Gruppe

einen Ring bilden können,
X Sauerstoff oder Schwefel
bedeuten, sowie deren herstellbare, pharmazeutisch akzeptablen Salze bzw. Säureadditionssalze.

Die Erfindung betrifft auch Verfahren zur Herstellung der Verbindungen der allgemeinen Formel I und ihrer Salze bzw. Säureadditionssalze sowie ihre Verwendung als pharmazeutische Wirkstoffe.

Für $R^1$, $R^2$ oder $R^3$ stehende Alkylreste haben bevorzugt 1 bis 3 Kohlenstoffatome. Besonders bevorzugt ist Methyl.

Die für $R^3$ oder $R^4$ stehenden Carbonsäuregruppierungen bedeuten bevorzugt Amide, wie zum Beispiel das unsubstituierte Amid, Mono- oder Dialkylamide mit 1 bis 3 Kohlenstoffatomen pro Alkylrest, Ester, wie zum Beispiel Alkyl ester mit 1 bis 4 Kohlenstoffatomen pro Alkylrest oder Benzylester, die Nitrilgruppe oder die Carboxygruppe selbst.

Alkylester sind bevorzugt der Methylester, der Ethylester und der tert.-Butylester.

Die für $R^5$ oder als Substituent einer für $R^4$ stehenden Carbonylgruppe stehenden gegebenenfalls

substituierten aliphatischen oder aromatischen 5- bis 7-gliedrigen Heterocyclen enthalten als Heteroglieder $>$O, $>$S, oder $>NR^7$, worin
$R^7$ Wasserstoff, Alkyl mit 1 bis 4, vorzugsweise 1 oder 2, Kohlenstoffatomen oder Alkoxycarbonyl mit 1 bis 4, vorzugsweise 1 oder 2, Kohlenstoffatomen im Alkoxyteil
bedeutet.

Sofern der Heterocyclus 2 Heteroglieder aufweist, können diese gleich oder verschieden sein. Ein Stickstoff enthaltender Heterocyclus kann auch über das Hetero-N-atom gebunden sein; er kann dann neben dem ersten, die Bindung vermittelnden Stickstoffatom noch ein beliebiges der oben genannten Heteroglieder enthalten. Beispiele für derartige, über ein Hetero-N-atom gebundene heterocyclische Reste sind der N-Pyrrolidinrest oder der N-Thiomorpholinrest.

Aromatische heterocyclische Reste sind solche, die aufgrund einer Konjugation von Doppelbindungen, gegebenenfalls mit freien Elektronenpaaren, innerhalb des Ringes mesomere Grenzstrukturen ausbilden können, wie z.B. der Thienylrest oder der Pyrazolylrest. Aliphatische heterocyclische Reste enthalten nur isolierte oder gar keine Doppelbindungen, wie z.B. der Pyrrolidinrest, der Piperidinrest, der Morpholinrest oder der Perhydrothiaze pinrest. Von den zwei Heteroglieder enthaltenden Heterocyclen sind solche bevorzugt, die mindestens ein Stickstoff enthaltendes Heteroglied aufweisen.

Beispiele für Heterocyclen, von denen sich für $R^5$ oder als Substituent einer für $R^4$ stehenden Carbonylgruppe stehenden heterocyclische Reste ableiten, sind: Thiophen, Di- oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, Imidazol, Imidazolin, Imidazolidin, Oxazol, Oxazolin, Oxazolidin, Thiazol, Thiazolin, Thiazolidin, Pyrimidin, Pyridazin, Pyrazin, Piperazin, Morpholin, Thiomorpholin, Diazepin, Oxazepin, Thiazepin, Perhydrodiazepin, -oxazepin und -thiazepin.

Besonders bevorzugte heterocyclische Reste leiten sich ab von Pyrrol, Pyrrolidin, Imidazol, Thiazol, Thiazolidin und Perhydrothiazepin.

Aliphatische heterocyclische Reste, insbesondere solche, die sich von Stickstoffheterocyclen ableiten, können auch an einem Ringkohlenstoffatom, vorzugsweise einem dem Stickstoff enthaltenden Heteroglied benachbarten Ringkohlenstoffatom, eine Ketofunktion, ein doppelt gebundenes Sauerstoffatom, aufweisen. Dieses kann auch in seiner tautomeren Form vorliegen.

Ein solcher, bevorzugter, Rest leitet sich beispielsweise vom 5-Oxo-perhydro-1,4-thiazepin ab.

Die heterocyclischen Reste können auch an einem der Ringkohlenstoffatome einen Substituenten wie z.B. eine Carboxylgruppe, eine Formylgruppe, Alkoxycarbonyl mit 1 bis 4, vorzugsweise 1 oder 2, Kohlenstoffatomen in der Alk oxygruppe oder, bevorzugt, eine Alkylgruppe mit 1 bis 4, vorzugsweise 1 oder 2, Kohlenstoffatomen tragen.

Die Alkylreste der für $R^4$ stehenden Alkylsulfinyl- und Alkylsulfonylgruppen haben bevorzugt 1 oder 2 Kohlenstoffatome.

Die Alkylreste der für $R^4$ stehenden Alkylcarbonyl- und Alkoxycarbonylvinylgruppen haben bevorzugt 1 bis 3 Kohlenstoffatome.

Für $R^5$ stehendes Alkyl hat bevorzugt 1 bis 5 und besonders bevorzugt 1 oder 2 Kohlenstoffatome und ist gegebenenfalls substituiert durch $-NH_2$, Monoalkylamino mit 1 bis 4, vorzugsweise 1 oder 2, Kohlenstoff-Atomen, Dialkylamino mit insgesamt 2 bis 6, vorzugsweise 2 bis 4, Kohlenstoff-Atomen, einen N-Pyrrolidinyl-, N-Piperidinyl-, N-Morpholinyl-, N-Thiomorpholinyl- oder durch einen in 4-Stellung gegebenenfalls durch Alkyl mit 1 bis 4 Kohlenstoff-Atomen, Phenyl, Toluyl, Chlorphenyl oder Methoxy- oder Ethoxyphenyl substituierten Piperazin-1-yl-Rest, durch Alkoxy mit 1 bis 4, vorzugsweise 1 oder 2, Kohlenstoff-Atomen oder durch gegebenenfalls substituiertes Phenoxy.

$R^6$ bedeutet bevorzugt Wasserstoff.

Ringe, die $R^5$ und $R^6$ zusammen mit der

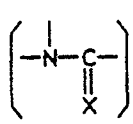 - Gruppe

bilden können, sind vorteilhafterweise 5- oder 6-gliedrig und können gegebenenfalls an einen Benzolkern kondensiert sein. Beispiele für solche Ringe sind 2-Pyrrolidinon, 2-Piperidinon, 3-Oxo-morpholin, 3-Oxothiomorpholin, Phthalimid, 2-Oxo-indolin, 1-Oxo-isoindolin und 4-Methyl-2-oxo-piperazin.

Besonders bevorzugt ist 2-Pyrrolidinon.

Ein für $R^4$ stehender Phenylsulfinyl-, Phenylsulfonyl-, Phenylcarbonyl- oder ein für $R^5$ stehender

Phenyl- oder Phenylamino-Rest sowie ein als Substituent an einen für $R^5$ stehenden Alkylrest gebundener Phenoxyrest kann im Kern seinerseits bis zu drei Substituenten tragen und zwar eine Aminogruppe, Monoalkylamino mit 1 bis 4, vorzugsweise 1 oder 2 Kohlenstoffatomen, Dialkylamino mit insgesamt 2 bis 6, vorzugsweise 2 bis 4, Kohlenstoffatomen, Alkanoylamino mit 1 bis 6, vorzugsweise 1 oder 2, Kohlenstoffatomen, Alkyl mit 1 bis 4, vorzugsweise 1 oder 2, Kohlenstoffatomen, Alkoxy mit 1 bis 4, vorzugsweise 1 oder 2 Kohlenstoffatomen, Halogen, vorzugsweise Fluor, Chlor oder Brom, insbesondere Fluor oder Chlor, Hydroxy, Nitro, Cyano, Carboxy oder Alkoxycarbonyl mit 1 bis 4, vorzugsweise 1 oder 2, Kohlenstoffatomen in der Alkoxygruppe. Als gegebenenfalls zweiter Substituent des Kerns kommen in Betracht: eine der oben definierten Alkyl- oder Alkoxygruppen oder eines der oben genannten Halogene und als dritter Substituent eine der obigen Alkyl- oder Alkoxygruppen.

Ein einzelner Substituent kann in 2-, 3- oder 4-Stellung des Phenylkerns stehen. Bei Zweifachsubstitution sind von den möglichen Positionen die 2,4-, die 3,4- und die 3,5-Stellung bevorzugt. Bei der Dreifachsubstitution kommen die Positionen 2,3,4 und 3,4,5 und 2,4,6 in Betracht.

Bevorzugte Substituenten für die genannten Phenylkerne sind Chlor, Alkyl und Alkoxy mit 1 oder 2 Kohlenstoffatomen, insbesondere Methyl oder Methoxy, Carboxy und Alkoxycarbonyl mit 1 oder 2 Kohlenstoffatomen in der Alkoxygruppe. Bevorzugt sind ferner neben den unsubstituierten Kernen die monosubstituierten und disubstituierten, welche als zweiten Substituenten einen Alkoxyrest tragen.

In einem an die Piperazingruppe gebundenen Toluyl-, Chlorphenyl- oder Alkoxyphenylrest können die Substituenten, bezogen auf den Piperazinrest, in 2-, 3- oder 4-Stellung, vorzugsweise in 2- oder 4-Stellung, stehen.

Spezielle, besonders bevorzugte Reste R sind Alkylreste mit 1 bis 3 Kohlenstoffatomen, die durch einen der folgenden Substituenten substituiert sind:
Formylamino, Acetylamino, Propionylamino, Isopropionylamino, Butyrylamino, 4-Chlorphenoxyacetylamino, N,N-Dimethylamino-acetylamino, L-Thiazolidin-4-yl-carbonylamino, 4-Chlorbenzoylamino, 5-Oxoperhydro-(1,4)-thiazepin-3-yl-carbonylamino, Aminocarbonylamino, 4-Chlorphenylaminocarbonylamino, 2-Oxopyrrolidin-1-yl.

Weitere wertvolle Substituenten eines für R stehenden Alkylrestes mit 1 bis 3 Kohlenstoffatomen sind z.B.: die Aminogruppe, 3,4-Dimethoxybenzoylamino, 2-(4-(2-Methoxyphenyl)-piperazinyl)-acetylamino, Isobutyrylamino, 4-Ethoxycarbonylphenylamino-carbonylamino, 4-Ethoxycarbonylphenylamino-thiocarbonylamino, 4-Carboxyphenylaminocarbonylamino, 4-Carboxyphenylamino-thiocarbonylamino.

In der allgemeinen Formel I können die Reste $R^1$, $R^2$, $R^3$ und $R^4$ an jeder beliebigen Position am Pyrrolkern sitzen.

Bevorzugt sitzt $R^4$ in 3-(bzw.4-)Stellung und $R^3$ in 4-(bzw.3-)Stellung. Die Reste $R^1$ und $R^2$ sitzen bevorzugt in 2- und 5-Stellung.

Die Herstellung der erfindungsgemäßen Verbindungen der allgemeinen Formel I kann beispielsweise durch elektrophile Substitution von Pyrrolen der allgemeinen Formel III

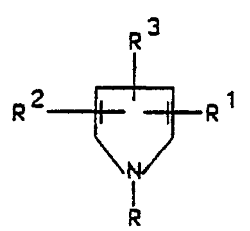

(III)

worin R, $R^1$, $R^2$ und $R^3$ die oben angegebenen Bedeutungen besitzen, mit Reagenzien, die Reste $R^4$ in den Ring einführen, erfolgen.

Derartige elektrophile Substitutionsreaktionen sind beispielsweise die Friedel-Crafts-Acylierung (zum Beispiel J. Org. Chem. 20, 230 (1955), Synth. Commun. 10, 773 (1980)), die Nitrierung (Org. Prep. Proced. Int. 19, 48 (1987)), die Sulfinylierung (J. Org. Chem. 45, 5336 (1980)), die vinyloge Vilsmeier-Formylierung (Synthesis 1983, 641) oder die Umsetzung mit Chlorsulfonylisocyanat (Synthesis 1985, 355).

Reagenzien, die durch elektrophile Substitution einen Rest $R^4$ in den Ring einführen, sind beispielsweise Carbonsäurechloride, Nitroniumionen, Sulfinsäurechloride, Dimethylaminoacrolein und Isocyanate.

Die bei der elektrophilen Substitution einzuhaltenden Reaktionsbedingungen, wie z.B. Temperatur, Lösungsmittel, Molverhältnisse, Katalysator, sind bekannt und können den oben zitierten Literaturstellen, aber auch gängigen Laboratoriumshandbüchern (z.B. Houben-Weyl) entnommen werden.

Die Verbindungen der allgemeinen Formel III sind bekannt und können entweder durch Umsetzung

entsprechend substituierter Furane mit Aminen der allgemeinen Formel IV

$H_2N$ - R (IV)

worin R die obengenannten Bedeutungen hat (analog US 2,655,512) oder durch Umsetzung von 1,4-Dicarbonylverbindungen mit Aminen der allgemeinen Formel IV (analog DE-A 35 27 791) erhalten werden.

Erfindungsgemäße Verbindungen der allgemeinen Formel I, in denen $R^4$ Dicyanmethylen, Alkoxycarbonylvinyl oder Nitril bedeutet, können auch hergestellt werden, indem man die entsprechenden Aldehydderivate mit Malonsäuredinitril (Org. Reactions 15, 232 (1967)), bzw. Malonsäureestern (Org. Reactions, 15, 229 (1967)) oder in einer Wittig-Reaktion (J. Org. Chem. 36, 1495 (1971), Chem. Ber. 88, 1654 (1955)) bzw. mit Hydroxylamin mit anschließender Wasserabspaltung (Synthesis 1982, 190) umsetzt.

Die genannten Aldehydderivate können durch Formylierung entsprechender Pyrrole der allgemeinen Formel III erhalten werden.

Als Formylierungsreaktionen eignen sich viele in der Literatur beschriebene Methoden (s. Houben-Weyl, Methoden der Organischen Chemie, 4. Auflage, E3, S. 16 ff. (1983)). In speziellen Fällen ist die Variante nach Reimer-Tiemann durch Umsetzung der Pyrrole mit Chloroform im alkalischen Medium angebracht, bessere Ausbeuten ergeben jedoch die Umsetzungen der Pyrrole mit 1,1-Dihalogenethern und Friedel-Crafts-Katalysatoren (J. Med. Chem. 15, 97 (1972)) oder mit Trialkoxymethanen und Trifluoressigsäure (J. Org. Chem 43, 282 (1978)). Am einfachsten führt die Vilsmeier-Haack-Reaktion durch Umsetzung der Pyrrole der allgemeinen Formel III mit Formamiden und Phosphoroxychlorid zu den erfindungsgemäßen Verbindungen (Methodicum Chimicum 5, S. 234 (1975), J. Org. Chem. 28, 3052 (1963)). Phosphoroxychlorid kann durch andere Ver bindungen wie Oxalylchlorid, Thionylchlorid, Sulfurylchlorid oder Cyanurchlorid ersetzt werden. Vorteilhaft wird die Reaktion in Gegenwart eines Lösungsmittels wie Dimethylformamid, 1,2-Dichlorethan oder Ethern durchgeführt. Auch Isonitrile in saurer Lösung eignen sich als Formylierungsagenzien für die erfindungsgemäßen Verbindungen (Chem. Ber. 94, 298 (1961)).

Die entsprechenden Aldehyde können aber auch direkt analog US 2,655,512 aus den Furanaldehyden und den entsprechenden Aminen erhalten werden.

Erfindungsgemäße Pyrrolcarbonsäurederivate können auch direkt durch Umsetzung aliphatischer 1,4-Dicarbonylverbindungen der allgemeinen Formel V

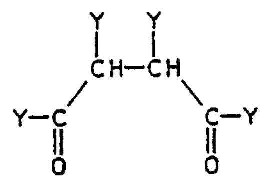

(V)

worin für Y die Reste $R^1$, $R^2$, $R^3$ und $R^4$ je nach gewünschtem Endprodukt stehen, mit Aminen der allgemeinen Formel IV erhalten werden. Eine Alternative zu dieser Umsetzung ist die Reaktion zwischen entsprechend substituierten 2-Halogenketonen, $\beta$-Ketoestern und primären Aminen der allgemeinen Formel IV (J. Am. Chem. Soc. 73 357 (1951)).

Üblicherweise werden diese Umsetzungen in geeigneten Lösungsmitteln bei Temperaturen von 20 bis 150°C, vorzugsweise unterhalb 100°C, insbesondere bei 40 bis 20°C, ausgeführt, wobei die Ausgangskomponenten normalerweise in etwa äquimolaren Mengen eingesetzt werden.

Gewünschtenfalls, z.B. bei Einsatz besonders niedrig siedender Lösungsmittel kann die Umsetzung auch unter Druck oberhalb des Siedepunktes des Reaktionsgemisches ausgeführt werden.

Geeignete Lösungsmittel sind z.B. Alkohole, insbesondere solche mit 1 bis 6 C-Atomen, wie z.B. Methanol, Ethanol, i- und n-Propanol, i-, sec- und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylether, Methylethylether, Di-n-propylether, Diisopropylether, Methyl-n-Butylether, Ethylpropylether, Dibutylether, Tetrahydrofuran, 1,4-Dioxan, 1,2-Dimethoxyethan, Bis-$\beta$-methoxyethylether; Polyether, wie z.B. Polyethylenglykole mit einem Molekulargewicht bis ca. 600; Oligoethylen-glykol-dimethylether, wie z.B. Pentaglyme; aliphatische Carbonsäuren, insbesondere Ameisen- und Essigsäure; Glykole und teilweise veretherte Glykole, wie z.B. Ethylenglykol, Propylenglykol, Trimethylenglykol, Ethylenglykol-monomethylether, Ethylenglykol-monoethylether, Diethylenglykol-monoethylether; aliphatische Kohlenwasserstoffe, wie z.B. niedrig-und hochsiedende Petrolether; aromatische Kohlenwasserstoffe, wie z.B. Benzol, Toluol, o-, m- und p-Xylol; halogenierte aliphatische oder aromatische Kohlenwasserstoffe, wie z.B. Methylenchlorid, Chloroform, Tetrachlorkohlenstoff, Chlorbenzol, Dichlorbenzol; Nitrile, wie z.B. Acetonitril; Amide, wie z.B. Dimethylformamid, N-Methyl-pyrrolidon; Hexamethylphosphorsäuretriamid; Sulfoxide, wie z.B. Dimethylsulfoxid; Wasser. Auch Gemische verschiedener Lösungsmittel können verwendet werden.

Das Amin der allgemeinen Formel IV kann auch in Form eines Säureadditionssalzes eingesetzt werden. Die Aufarbeitung der Ansätze erfolgt nach üblichen Methoden. Die Umsetzung kann gegebenenfalls auch in Anwesenheit einer Base oder eines Basengemisches durchgeführt werden. Geeignete Basen sind z.B. tertiäre aliphatische Amine, wie z.B. Triethylamin, Tri-n-propylamin und Tri-iso-propylamin, ferner Pyridin, sowie Alkali-carbonate, -hydrogencarbonate.

Die Amine der allgemeinen Formel IV sind bekannt bzw. lassen sich leicht nach den für die Herstellung primärer Amine üblichen Methoden herstellen.

Auch die 1,4-Dicarbonylverbindungen der allgemeinen Formel V sind bekannt und/oder nach bekannten Verfahren herstellbar, so zum Beispiel nach Stetter, Angew. Chem. 88 695 (1976).

Durch Abspaltung des Acylrestes aus erfindungsgemäßen Verbindungen der allgemeinen Formel I, in denen R ein durch die Gruppe der allgemeinen Formel II

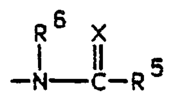 (II)

substituierter Alkylrest ist, können die entsprechenden erfindungsgemäßen Verbindungen, in denen der für R stehende Alkylrest durch eine primäre Aminogruppe substituiert ist, erhalten werden. Die Abspaltung wird in an sich bekannter Weise hydrolytisch durchgeführt. Hierzu werden die Verbindungen mit Wasser oder einem wasserhaltigen organischen Medium in Gegenwart von molaren Mengen einer Base behandelt. Die Behandlungsdauer hängt von der gewählten Temperatur ab. Man kann bei Zimmertemperatur oder, um die Hydrolyse zu beschleunigen, bei erhöhter Temperatur, zweckmäßigerweise bis zur Rückflußtemperatur des flüssigen Hydrolysemediums, arbeiten.

Die Art der zuzusetzenden Base ist im Prinzip belanglos. Diese Reagentien sollen nur eine ausreichend hohe $OH^{\ominus}$-Konzentration herbeiführen und eine einfache Aufarbeitung der Ansätze gestatten. Die Wahl dieser Mittel kann daher in bekannter Weise erfolgen.

Zur Herstellung der erfindungsgemäßen Verbindungen der allgemeinen Formel I, in denen R ein durch eine Acylaminogruppe der allgemeinen Formel IIa

$-NH-\overset{\overset{X}{\|}}{C}-R^5$ (IIa)

substituierter Alkylrest ist, können auch erfindungsgemäße Aminoalkylpyrrole der allgemeinen Formel Ia

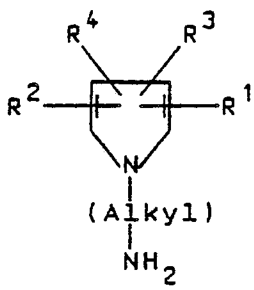 (Ia)

mit reaktiven Carbonsäurederivaten, abgeleitet von Carbonsäuren der allgemeinen Formel VI

$R^5$-COOH (VI)

worin $R^5$ die oben angegebene Bedeutung hat oder auch mit Alkalicyanat bzw. -thiocyanat oder mit Isocyanaten bzw. Isothiocyanaten der allgemeinen Formel VII

$R^7$-NCZ (VII)

worin Z Sauerstoff oder Schwefel ist und $R^7$ Alkyl mit 1 bis 5 Kohlenstoffatomen oder Phenyl bedeutet, das gegebenenfalls in der oben präzisierten Weise substituiert ist, acyliert werden.

Als reaktive Carbonsäurederivate eignen sich Carbonsäureester, Carbonsäureanhydride, Carbonsäurechloride oder Carbonsäuren, die in situ aktiviert werden, wie z.B. mit Dicyclohexylcarbodiimid (Houben Weyl 8, 522); Oxalylchlorid (GB 2139-225); N,N-Carbonyldiimidazol (J.Med.chem. 1982, 620; Synthesis 1982, 833; Chem.Pharm. Bull. 32, 5044 (1984)); N,N'-Carbonyldiazolen (bull. Chem. Soc. Jap. 57, 3597 (1984)); Kohlensäure-di-(2-pyridyl)-ester (Tetrahedron Lett. 25, 4943 (1983)); Chlorameisensäureester (Tetrahedron Lett. 24, 3365 (1983)); Diphosphortetrajodid (Chem. Lett. 1983, 449); Dialkyldisulfit (Indian J. Chem. 21, 259 (1982)); Methylethylphosphinsäureanhydrid oder mit anderen reaktiven Agenzien.

Bei Einsatz von Isocyanaten bzw. Isothiocyanaten der Formeln $R^7$-NCO bzw. $R^7$-NCS als Acylierungs-

mittel werden diejenigen erfindungsgemäßen Pyrrolderivate erhalten, in denen R ein durch die Gruppen -HN-CO-NH-$R^7$ bzw. -NH-CS-NH-$R^7$ substituierter Alkylrest ist. Umsetzung der erfindungsgemäßen Verbindungen der allgemeinen Formel Ia mit Alkalicyanaten bzw. -thiocyanaten liefert erfindungsgemäße Verbindungen, in denen R ein durch die Gruppen -NH-CO-$NH_2$ bzw. -NH-CS-$NH_2$ substituierter Alkylrest ist.

Die Umsetzungen werden zweckmäßigerweise in flüssiger Phase durchgeführt, wobei die Anwesenheit eines inerten Lösungsmittels vorteilhaft ist.

Werden enantiomerenreine Carbonsäurederivate oder erfindungsgemäße Verbindungen der allgemeinen Formel Ia eingesetzt, so können auch die erfindungsgemäßen Verbindungen der Formel I als enantiomerenreine Verbindungen erhalten werden.

Sofern die erfindungsgemäßen Verbindungen der allgemeinen Formel I basische Reste enthalten, bilden sie mit anorganischen oder organischen Säuren Säureadditionssalze. Zur Bildung derartiger Säureadditionssalze sind anorganische und organische Säuren geeignet. Geeignete Säuren sind beispielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Nicotin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze werden wie üblich durch Vereinigung der Komponenten, zweckmäßigerweise in einem geeigneten Lösungsmittel oder Verdünnungsmittel, hergestellt. Bei der Synthese der Verbindungen der Formel I können zunächst die Säureadditionssalze im Zuge der Aufarbeitung anfallen. Aus den Säureadditionssalzen können die freien Verbindungen der allgemeinen Formel I gewünschtenfalls in bekannter Weise, z.B. durch Auflösen oder Suspendieren in Wasser und Alkalischstellen, z.B. mit Natronlauge, und anschließendes Isolieren, gewonnen werden.

Verbindungen der allgemeinen Formel I, in denen für $R^3$ und/oder $R^4$ eine Carboxylgruppe steht, können auch in Salzform vorliegen. Bevorzugt sind die Natrium-, Kalium-und Ammoniumsalze, die durch Umsetzung der sauren Form mit entsprechenden Basen erhalten werden können. Falls die Verbindungen der allgemeinen Formel I neben einer Carboxygruppe auch eine freie Aminogruppe im Rest R enthalten, können sie auch in Form innerer Salze vorliegen.

Die erfindungsgemäßen Verbindungen der allgemeinen Formel I und ihre pharmazeutisch akzeptablen Säureadditionssalze besitzen wertvolle pharmakologische Eigenschaften. Sie sind zentralwirksam, beispielsweise zeigen sie encephalotrope und nootrope Wirkungen und dienen zur Behandlung von Krankheiten der Gehirnfunktionen wie cerebraler Insuffizienz, cerebraler Alterungsvorgänge, verminderter Gedächtnisleistung, wie sie auch bei der Alzheimer Krankheit oder der Multi-Infarkt Demenz oder bei verminderter Lernleistung auftreten. Sie sind den bisher bekannten Verbindungen gleicher Wirkungsrichtung überraschenderweise erheblich überlegen. Sie zeigen eine ausgezeichnete Wirksamkeit in verschiedenenartigen Tests, wie z B. bei der Verlängerung der Überlebenszeit unter Natriumnitrit-Hypoxie nach Gibsen und Bless (J. Neurochemistry 27, (1976)), bei der Verbesserung der stickstoffinduzierten Hypoxietoleranz, wobei Versuchstiere nach Prämedikation mit den untersuchten Präparaten mit reinem Stickstoff beatmet werden und die Verlängerung des Zeitraumes zwischen Einsatz der Beatmung und elektrischer Neutralität des Elektroencephalogramms sowie die Letalität gemessen werden.
Auch in Tests, die direkt auf die Erfassung der Lern- und Gedächtnisleistung abzielen, wie z.B. den bekannten "avoidance"-Tests, sind die erfindungsgemäßen Produkte sehr gut wirksam.

Die Prüfung in den genannten und einer Reihe weiterer Tests, wie z.B. dem $\gamma$-Butyrolacton-Test, ergibt, daß die erfindungsgemäßen Verbindungen in niedrigen Dosen bei geringer Toxizität überraschenderweise ein besonders günstiges, bei bekannten Präparaten in dieser Form nicht vorliegendes Wirkprofil aufweisen.

Die Verbindungen der allgemeinen Formel I und ihre physiologisch verträglichen Salze stellen somit eine Bereicherung der Pharmazie dar.

Die erfindungsgemäßen Verbindungen der allgemeinen Formel I und die vorgenannten Verbindungen und ihre pharmazeutisch akzeptablen Säureadditionssalze können daher am Menschen als Heilmittel z.B. bei der Bekämpfung bzw. Vorbeugung von Erkrankungen, die durch eine Einschränkung der Gehirnfunktion bedingt sind und bei der Behandlung und Vorbeugung von cerebralen Alterungsvorgängen Anwendung finden.

Die Verbindungen der allgemeinen Formel I und die vorgenannten Verbindungen und ihre pharmazeutisch akzeptablen Säureadditionssalze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I bzw. der vorgenannten Verbindungen oder eines Säureadditionssalzes davon, neben üblichen pharmazeutisch einwandfreien Träger- und Zusatzstoffen enthalten. Die Zubereitungen enthalten normalerweise etwa 0,5 bis 90 Gew.% der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glycerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Ver bindungen, wie Digoxin, Acetyldigoxin, Metildigoxin und Lanato-Glykoside; Coronardilatatoren, wie Carbocromen, Dipyridamol, Nifedipin und Perhexilin, antianginöse Verbindungen, wie Isosorbiddinitrat, Isosorbidmononitrat, Glycerolnitrat, Molsidomin und Verapamil, β-Blocker, wie Propranolol, Oxprenolol, Atenolol, Metoprolol und Penbutolol. Darüber hinaus lassen sich die Verbindungen mit anderen nootrop wirksamen Substanzen, wie z.B. Piracetam, oder ZNS-aktiven Substanzen, wie Pirlindol, Sulpirid etc. kombinieren.

Die Dosierung kann innerhalb weiter Grenzen variieren und ist in jedem einzelnen Fall den individuellen Gegebenenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Application beträgt die Tagesdosis im allgemeinen etwa 0,01 bis 0,3 mg/kg, vorzugsweise 0,05 bis 0,1 mg/kg KörPergewicht. Die Tagesdosis wird normalerweise, insbesondere bei der Applikation größerer Mengen, in mehrere, z.B. 2, 3 oder 4, Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,1 bis 50 mg, vorzugsweise 0,5 bis 10 mg Wirkstoff der allgemeinen Formel I oder eines pharmakologisch annehmbaren Salzes davon pro Dosis.

Die folgenden Beispiele 1 bis 31 betreffen die Herstellung von Verbindungen der allgemeinen Formel I, die Beispiele A bis H betreffen die Herstellung von Zubereitungen der Verbindungen der allgemeinen Formel I.

Beispiel 1:

1-(2-Acetylaminoethyl)-2,4-dimethylpyrrol-3-carbonsäuremethylester

Zu 40,7 g (0,35 mol) Acetessigsäuremethylester werden langsam 71,5 g (0,70 mol) (2-Acetylaminoethyl)-amin zugegeben. Anschließend tropft man 32,4 g (0,35 mol) Chloraceton zu und erhitzt 20 Stunden unter Rückfluß. Nach dem Abkühlen wird Wasser zugesetzt, mit Methylenchlorid extrahiert und eingeengt. Der Rückstand wird aus Essigsäureethylester (über Aktivkohle und Kieselgel filtriert) umkristallisiert.
Ausbeute: 37,0 g (44 % d.Theorie)
Schmelzpunkt: 130 - 132°C
Elementaranalyse: $C_{12}H_{18}N_2O_3$ (238,29)

| | | | | |
|---|---|---|---|---|
| Ber.: | C 60,5 | H 7,6 | N 11,8 | O 20,1 |
| Gef.: | C 60,8 | H 7,7 | N 11,9 | O 19,8 |

Beispiel 2:

1-(2-Acetylaminoethyl)-2,4-dimethylpyrrol-3-carbonsäurebenzylester

Analog Beispiel 1 erhält man durch Umsetzung von Chloraceton, 2-Acetylaminoethylamin und Acetessigsäurebenzylester.
Schmelzpunkt: 115 - 117°C

Beispiel 3:

1-(2-Acetylaminoethyl)-2,5-dimethylpyrrol-3-carbonsäurebenzylester

7,1 g (0,029 mol) Acetonylacetessigsäurebenzylester und 3,0 g (0,029 mol) 2-(Acetylaminoethyl)-amin werden in 100 ml Isopropanol 2 h unter Rückfluß erhitzt. Man engt ein, nimmt in verdünnter Salzsäure auf und extrahiert mit Methylenchlorid. Das erhaltene Öl (8,3 g) wird über eine Kieselgelsäule mit Methylenchlorid chromatographiert und aus Dibutylether umkristallisiert.
Ausbeute: 5,1 g (56 % d. Theorie)
Schmelzpunkt: 82 - 84°C.

Beispiel 4:

1-(2-Acetylaminoethyl)-2,4,5-trimethylpyrrol-3-carbonsäuremethylester

Durch Umsetzung von Acetessigsäuremethylester mit 3-Chlor-2-butanon und 2-Acetylaminoethylamin analog Beispiel 1.
Schmelzpunkt: 163 - 165°C

Beispiel 5:

1-(2-Acetylaminoethyl)-2,4,5-trimethylpyrrol-3-carbonsäure-tert-butylester

Durch Reaktion von Acetessigsäure-tert-butylester mit 3-Chlor-2-butanon und 2-Acetylaminoethylamin analog Beispiel 1.
Schmelzpunkt: 159 - 160°C

Beispiel 6:

1-(2-Acetylaminoethyl)-2,5-dimethylpyrrol-3-carbonsäure

2,4 g (7,6 mmol) 1-(2-Acetylaminoethyl)-2,5-dimethylPyrrol-3-carbonsäurebenzylester aus Beispiel 3 werden in 200 ml Ethanol gelöst und in Gegenwart von 0,5 g 10% Pd-Kohle bei Raumtemperatur hydriert. Nach dem Einengen wird der erhaltene Feststoff mit Methylenchlorid verrührt und abgesaugt.
Schmelzpunkt: 223 - 224°C

Beispiel 7:

1-(2-Acetylaminoethyl)-2,4-dimethylpyrrol-3-carbonsäure

Analog Beispiel 6 erhält man aus 1-(2-Acetylaminoethyl)-2,4-dimethylpyrrol-3-carbonsäurebenzylester (Beispiel 2) die entsprechende Säure.
Schmelzpunkt: 165 - 167°C

Analog der bisher angegebenen Verfahren lassen sich folgende weitere erfindungsgemäße Verbindungen herstellen:

Beispiel 8

1-(3-Acetylaminopropyl)-2,5-dimethylpyrrol-3-carbonsäuremethylester,

Beispiel 9

1-(4-Acetylaminobutyl)-2,5-dimethylpyrrol-3-carbonsäureethylester,

Beispiel 10

1-(2-Propionylamino-1-methylethyl)-pyrrol-2-carbonsäurepropylester

Beispiel 11

1-(2-Formylaminoethyl)-pyrrol-3-carbonsäuremethylester

Beispiel 12

1-(2-Benzoylaminopropyl)-2,5-dimethylpyrrol-3-carbonsäurebenzylester.

Beispiel 13:

1-(2-Acetylaminoethyl)-2,5-dimethylpyrrol-3-carbonsäurenitril

5,2 g (0,025 mol) 1-(2-Acetylaminoethyl)-2,5-dimethylpyrrol-3-aldehyd, 1,75 g (0,025 mol) Hydroxylaminohydro chlorid und 2 ml (0,025 mol) Pyridin werden in 25 ml Toluol 1,5 h unter Rückfluß erhitzt. Nach dem Einengen wird in Wasser aufgenommen, mit Methylenchlorid extrahiert und aus Toluol umkristallisiert.
Ausbeute: 3,45 g (67 % d.Theorie)
Schmelzpunkt: 131 - 133°C
Elementaranalyse: $C_{11}H_{15}N_3O$ (205,26)

| | | | | |
|---|---|---|---|---|
| Ber.: | C 64,4 | H 7,4 | N 7,8 | O 20,5 |
| Gef.: | C 64,3 | H 7,1 | N 8,0 | O 20,5 |

Die Vorstufe wird auf folgendem Wege erhalten:
Zu 13 ml (0,168 mol) wasserfreiem Dimethylformamid werden bei 0°C langsam 15,5 ml (0,166 mol) Phosphoroxychlorid zugetropft. Man rührt 15 min bei 10°C, gibt 70 ml 1,2-Dichlorethan zu und tropft anschließend 29,4 g (0,163 mol) 1-(2-Acetylaminoethyl)-2,5-dimethylpyrrol in 25 ml 1,2-Dichlorethan bei 5°C. Man rührt 6 Stunden bei Raumtemperatur, gibt 79,5 g (0,97 mol) Natriumacetat in 130 ml Wasser zu, erhitzt 15 min unter Rückfluß, trennt die Phasen und engt die organische Phase ein. Der Rückstand wird mit Toluol ausgekocht und das ausfallende Produkt abfiltriert und aus Essigester umkristallisiert.

Beispiel 14:

1-(2-Acetylaminoethyl)-2,5-dimethylpyrrol-3-yl-methylenmalonsäuredinitril

4,2 g (0,02 mol) 1-(2-Acetylaminoethyl)-2,5-dimethylpyrrol-3-aldehyd (siehe Beispiel 13) und 1,35 g (0,02 mol) Malonsäuredinitril werden in 40 ml Ethanol gelöst. Nach Zugabe von 1 Tropfen 10proz. Kaliumhydroxidlösung wird 20 h bei Raumtemperatur gerührt, abfiltriert und aus Isopropanol umkristallisiert.
Ausbeute: 3,1 g (60 % d.Theorie)
Schmelzpunkt: 184 - 185°C
Elementaranalyse: $C_{14}H_{16}N_4O$ (256,31)

| | | | | |
|---|---|---|---|---|
| Ber.: | C 65,6 | H 6,3 | N 21,9 | O 6,2 |
| Gef.: | C 65,4 | H 6,4 | N 21,7 | O 6,6 |

Beispiel 15:

3-(1-(2-Acetylaminoethyl)-2,5-dimethylpyrrol-3-yl)-2-propensäuremethylester

6,1 g (0,05 mol) Malonsäuremonomethylester werden in 20 ml Pyridin gelöst. Nach Zugabe von 10,4 g (0,05 mol) 1-(2-Acetylaminoethyl)-2,5-dimethylpyrrol-3-aldehyd (siehe Beispiel 13) und 1 ml Piperidin wird 15 h unter Rückfluß erhitzt. Nach Zugabe von weiteren 27 g Malonsäuremonomethylester wird 24 h weiter erhitzt, eingeengt, in Wasser aufgenommen, mit Methylenchlorid extrahiert, die Methylenchloridphase mit verdünnter Säure und Lauge gewaschen und eingeengt. Nach Säulenchromatographie mit Methylenchlorid : Methanol = 98 : 2 als Laufmittel erhält man 7,5 g Produkt, das aus Essigsäureethylester umkristallisiert werden kann.
Ausbeute: 6,5 g (49 % d.Theorie)
Schmelzpunkt: 144 - 145°C
Elementaranalyse: $C_{14}H_{20}N_2O_3$ (264,32)

| | | | | |
|---|---|---|---|---|
| Ber.: | C 63,6 | H 7,6 | N 10,6 | O 18,2 |
| Gef.: | C 63,9 | H 7,1 | N 10,3 | O 18,4 |

Beispiel 16:

1-(2-Acetylaminoethyl)-2,5-dimethylpyrrol-3,4-dicarbonsäuredinitril

9,0 g (0,05 mol) 1-(2-Acetylaminoethyl)-2,5-dimethyl pyrrol werden in 50 ml Acetonitril gelöst. Nach Zutropfen von 10,5 ml (0,12 mol) Chlorsulfonylisocyanat in 15 ml Acetonitril bei -10°C wird 12 h bei Raumtemperatur nachgerührt und bei 0°C 4,2 ml Dimethylformamid zugetropft. Nach 1 h bei 50°C wird auf Eis gegossen, mit Methylenchlorid extrahiert, eingeengt und über eine kurze Kieselgelsäule chromatographiert.
Ausbeute: 2,3 g (20 % d.Theorie)
Schmelzpunkt: 197 - 199°C Elementaranalyse: $C_{12}H_{14}N_4O$ (230,27)

| | | | | |
|---|---|---|---|---|
| Ber.: | C 62,6 | H 6,1 | N 24,3 | O 6,9 |
| Gef.: | C 62,2 | H 6,3 | N 24,1 | O 7,5 |

Beispiel 17:

3-(1-(2-Acetylaminoethyl)-2,5-dimethylpyrrol-3-yl)-2-propenaldehyd

16,2 g (0,09 mol) 1-(2-Acetylaminoethyl)-2,5-dimethylpyrrol und 10 g (0,1 mol) 3-Dimethylaminoacrolein werden in 80 ml 1,2-Dichlorethan gelöst. Bei -10°C werden 9 ml (0,098 mol) Phosphoroxychlorid zugetropft. Nach 20 Min. werden 41 g (0,5 mol) Natriumacetat in 150 ml Wasser zugegeben, 18 Stunden bei Raumtemperatur gerührt, 2 h auf 70°C erwärmt, die Phasen getrennt, die organische Phase über eine Kieselgelsäule chromatographiert und das Produkt aus Isopropanol umkristallisiert.
Ausbeute: 2,3 g (11 % d.Theorie)
Schmelzpunkt: 186 - 187°C
Elementaranalyse: $C_{13}H_{18}N_2O_2$ (234,30)

| | | | | |
|---|---|---|---|---|
| Ber.: | C 66,6 | H 7,7 | N 12,0 | O 13,7 |
| Gef.: | C 66,4 | H 7,8 | N 11,7 | O 13,8 |

Beispiel 18:

1-(2-Acetylaminoethyl)-2,5-dimethyl-3-nitropyrrol

18,0 g (0,1 mol) 1-(2-acetylaminoethyl)-2,5-dimethylPyrrol werden bei -10°C in 54 ml konz. Schwefelsäure eingetragen, wobei die Temperatur auf 9°C ansteigt. Nach 5 Minuten nachrühren werden bei etwa 5°C 10,9 g (0,11 mol) Kaliumnitrat eingetragen. Nach 30 Minuten weiterrühren wird auf 300 g Eis gegossen, mit Methylenchlorid extrahiert, eingeengt und über eine kurze Kieselsäule chromatographiert.
Ausbeute: 4,3 g (19 % d.Theorie)
Schmelzpunkt: 155 - 157°C Elementaranalyse: $C_{10}H_{15}N_3O_3$ (225,25)

| | | | | |
|---|---|---|---|---|
| Ber.: | C 53,3 | H 6,7 | N 18,7 | O 21,3 |
| Gef.: | C 53,7 | H 6,5 | N 19,0 | O 21,3 |

Beispiel 19:

(1-(2-Acetylaminoethyl)-2,5-dimethyl-3-trifluoracetylpyrrol

Zu 7 ml (0,05 mol) Trifluoressigsäureanhydrid in 10 ml Toluol werden 8,1 g (0,045 mol) 1-(2-Acetylaminoethyl)-2,5-dimethylpyrrol in 60 ml Toluol bei -5°C zugetropft. Nach 20 h bei Raumtemperatur wird mit Natriumhydrogencarbonatlösung hydrolysiert, abgesaugt und aus Ligroin umkristallisiert.
Ausbeute: 6,8 g (55 % d.Theorie)
Schmelzpunkt: 128 - 130°C
Elementaranalyse: $C_{12}H_{15}N_2O_2F_3$ (276,26)

| | | | | |
|---|---|---|---|---|
| Ber.: | C 52,2 | H 5,5 | N 10,1 | F 20,6 |
| Gef.: | C 51,9 | H 5,3 | N 9,9 | F 21,0 |

Beispiel 20:

3-Acetyl-1-(2-acetylaminoethyl)-2,5-dimethylpyrrol

13,7 g (0,1 mol) wasserfreies Zinkchlorid werden in 100 ml Ether gelöst und zu 18,0 g (0,1 mol) 1-(2-Acetylaminoethyl)-2,5-dimethylpyrrol und 9,5 ml (0,1 mol) Essigsäureanhydrid in 50 ml Ether zugegeben. Nach 1 h bei Raumtemperatur werden 50 ml Ethylenglykoldimethylether zugegeben und 20 Stunden weitergerührt. Nach der Hydrolyse wird die organische Phase eingeengt, chromatographiert und aus Essigsäureethylester umkristallisiert.
Ausbeute: 4,5 g (20 % d.Theorie)
Schmelzpunkt: 136 - 138°C
Elementaranalyse: $C_{12}H_{18}N_2O_2$ (222,29)

| | | | | |
|---|---|---|---|---|
| Ber.: | C 64,8 | H 8,2 | N 12,6 | O 14,4 |
| Gef.: | C 65,0 | H 8,0 | N 12,5 | O 14,5 |

Beispiel 21

1-(2-Acetylaminoethyl)-3-benzoyl-2,5-dimethyl-pyrrol

Analog Beispiel 20 mit Benzoesäureanhydrid statt Essigsäureanhydrid.
Schmelzpunkt: 152 - 154°C

Beispiel 22:

1-(2-Acetylaminoethyl)-2,5-dimethyl-3-(2-pyrrolcarbonyl)pyrrol

Durch Acylierung mit Pyrrol-2-carbonsäurechlorid analog Beispiel 20.
Schmelzpunkt: 169 - 171°C

Beispiel 23:

1-(2-Acetylaminoethyl)-2,5-dimethyl-3-methylsulfinylpyrrol

19,7 g (0,2 mol) Methylsulfinylchlorid werden in 500 ml Methylenchlorid gelöst. Bei 0°C tropft man 36,0 g (0,2 mol) 1-(2-Acetylaminoethyl)-2,5-dimethylpyrrol und 27,6 ml (0,2 mol) Triethylamin in 500 ml Methylenchlorid zu, rührt 45 Min. bei 0°C und gießt auf wäßrige Natriumhydrogencarbonatlösung. Die organische Phase wird abgetrennt und verworfen. Die Wasserphase wird eingeengt, der Rückstand mit Isopropanol ausgekocht und nach dem Einengen chromatographiert.
Ausbeute: 9,4 g (19 % d.Theorie)
Schmelzpunkt: 113 - 115°C
Elementaranalyse: $C_{11}H_{18}N_2O_2S$ (242,34)

| | | | | |
|---|---|---|---|---|
| Ber.: | C 54,5 | H 7,5 | N 11,6 | O 13,2 |
| Gef.: | C 54,7 | H 7,2 | N 11,5 | O 13,3 |

Analog den oben angegebenen Beispielen lassen sich weitere erfindungsgemäße Verbindungen herstellen, beispielsweise:

Beispiel 24

1-(3-Acetylaminopropyl)-2,5-dimethylpyrrol-3-carbonsäurebenzylester

Schmelzpunkt: 75 - 76°C

Beispiel 25

1-(2-Butyrylaminoethyl)-2,5-dimethylpyrrol-3-carbonsäurebenzylester
Schmelzpunkt: 92 - 93°C

Beispiel 26

2,5-Dimethyl-1-(2-(2-oxo-pyrrolidin-1-yl)-ethyl)-pyrrol-3-carbonsäurenitril

Beispiel 27

1-(2-Formylaminoethyl)-2,5-diethyl-3-phenylsulfonylpyrrol

Beispiel 28

1-(4-Aminocarbonylamino-butyl)-2,5-dimethylpyrrol-3-carbonsäure-tert-butylester

Beispiel 29

1-(2-(5-Oxo-perhydro-1,4-thiazepin-3-carbonyl)-amino)-ethyl-2-propyl-pyrrol-5-carbonsäurenitril

Beispiel 30

1-(2-(3,4-Dimethoxybenzoylamino)-ethyl)-2,5-dimethylpyrrol-3-carbonsäureethylester

Beispiel 31

1-(2-Acetylaminoethyl)-2,5-dimethylpyrrol-3-carbonsäureamid
Schmelzpunkt: 180-182°C

Beispiel 32

1-(2-Acetylaminoethyl)-2,5-dimethylpyrrol-3-carbonsäure tert.-butylester
Schmelzpunkt: 124-125°C

Beispiel 33

2,5-Dimethyl-1-(2-(2-oxopyrrolidin-1-yl)-ethyl)-pyrrol-3-carbonsäure
Schmelzpunkt: 199-201°C

Beispiel 34

1-(2-Aminoethyl)-2,5-dimethylpyrrol-3-carbonsäure
Schmelzpunkt: 195-196°C

Beispiel 35

1-(2-Benzyloxycarbonylamino-ethyl)-2,5-dimethylpyrrol-3-carbonsäurebenzylester
Schmelzpunkt: 96-98°C

Beispiel 36

1-(2-n-Butyrylamino-ethyl)-2,5-dimethylpyrrol-3-carbonsäure
Schmelzpunkt: 170-171°C

Beispiel 37

1-(3-Acetylaminopropyl)-2,5-dimethylpyrrol-3-carbonsäure
Schmelzpunkt: 175-176°C

Beispiel 38

1-(3-Acetylaminopropyl)-2,5-dimethylpyrrol-3-carbonsäureamid
Schmelzpunkt 162-165°C

Beispiel A

Emulsionen mit 3 mg Wirkstoff per 5ml können nach folgender Rezeptur hergestellt werden

| | |
|---|---|
| Wirkstoff | 0.06g |
| Neutralöl | q.s. |
| Natriumcarboxymethylzellulose | 0,6g |
| Polyoxyethylenstearat | q.s. |
| Reinglyzerin | 0,2 bis 2g |
| Aromastoffe | q.s. |
| Wasser (entmineralisiert oder destilliert) | ad 100ml |

Beispiel B

Tabletten können nach folgender Formulierung hergestellt werden:

| | |
|---|---|
| Wirkstoff | 2mg |
| Lactose | 60mg |
| Maisstärke | 30mg |
| lösliche Stärke | 4mg |
| Magnesiumstearat | 4mg |
| | 100mg |

Beispiel C

Für die Herstellung von Weichgelatinekapseln mit 5mg Wirkstoff pro Kapsel eignet sich die folgende Zusammensetzung

| | |
|---|---|
| Wirkstoff | 5mg |
| Mischung von Triglyzeriden aus Kokosöl | 150mg |
| Kapselinhalt | 155mg |

Beispiel D

Für die Herstellung von Dragees eignet sich folgende Formulierung:

| | |
|---|---|
| Wirkstoff | 3mg |
| Maisstärke | 100mg |
| Lactose | 55mg |
| sec Calciumphosphat | 30mg |
| lösliche Stärke | 3mg |
| Magnesiumstearat | 5mg |
| kolloidale Kieselsäure | 4mg |
| | 200mg |

Beispiel E

Dragees, enthaltend einen erfindungsgemäßen Wirkstoff und einen anderen therapeutisch wirksamen Stoff:

| | |
|---|---|
| Wirkstoff | 6mg |
| Propranolol | 40mg |
| Milchzucker | 90mg |
| Maisstärke | 90mg |
| sec Calciumphosphat | 34mg |
| lösliche Stärke | 3mg |
| Magnesiumstearat | 3mg |
| kolloidale Kieselsäure | 4mg |
| | 270mg |

Beispiel F

Dragees, enthaltend einen erfindungsgemäßen Wirkstoff und einen anderen therapeutisch wirksamen Stoff:

| | |
|---|---|
| Wirkstoff | 5mg |
| Pirlindol | 5mg |
| Milchzucker | 60mg |
| Maisstärke | 90mg |
| sec Calciumphosphat | 30mg |
| lösliche Stärke | 3mg |
| Magnesiumstearat | 3mg |
| kolloidale Kieselsäure | 4mg |
| | 200mg |

Beispiel G

Kapseln, enthaltend einen erfindungsgemäßen Wirkstoff und einen anderen therapeutisch wirksamen Stoff:

| | |
|---|---|
| Wirkstoff | 5mg |
| Nicergolin | 5mg |
| Maisstärke | 185mg |
| | 195mg |

## Tabelle 1

Prozentuale Umkehr der Störung der Haltefähigkeit nach Verabreichung von 175 mg/kg s.c. $NaNO_2$ und Praemedikation mit den Verbindungen der allgemeinen Formel I.

| Verbindungen der allg. Formel I gemäß Beispiel | Dosis mg/kg (p.o.) | Prozentuale Umkehr des Hypoxie-Effektes |
|---|---|---|
| 1 | 0,3 i.p. | 61 |
| 3 | 3 | 62 |
| 4 | 3 | 37 |
| 5 | 3 | 91 |
| 6 | 3 | 57 |
| 13 | 3 | 49 |
| 14 | 3 | 40 |
| 15 | 0,3 | 53 |
| 16 | 1 | 59 |
| 17 | 3 | 30 |
| 18 | 0,3 | 33 |
| 19 | 0,3 | 48 |
| 20 | 0,3 | 55 |
| 21 | 0,3 | 47 |
| 23 | 3 | 75 |
| 24 | 30 | 63 |
| 25 | 3 | 53 |
| 32 | 10 | 62 |
| 33 | 30 | 45 |
| 35 | 3 | 38 |
| 36 | 3 | 61 |
| 37 | 3 | 80 |
| 38 | 30 | 40 |
| vgl. Piracetam | 10 | 19 |

"Passive avoidance"

Die Testapparatur ist eine Hell-Dunkel-Box mit elektrifizierbarem Gitterboden im dunklen Teil.

55 Minuten nach Verabreichung von Kontroll- und Präparatinjektion werden unerfahrene männliche Mäuse mit Scopolaminhydrobromid (3 mg/kg i.p.) behandelt. 5 Minuten später werden die Mäuse in den hellen Teil der Box gesetzt. Nach dem Überwechseln in den dunklen Teil der Box erhalten sie einen ihnen unangenehmen elektrischen Fußschock. Nach 24 Stunden wird jede Maus einmal in den hellen Teil der Testapparatur gesetzt und die Verweildauer (max. 180 sec.) gemessen. Die mit einer aktiven Dosis eines Präparats und Scopolamin behandelten Tiere zeigen eine lange Verweildauer, ebenso die nicht mit Scopolamin behandelten Tiere, wogegen diejenigen mit Kontrollinjektion und Scopolamin behandelten eine kurze Verweildauer zeigen. Die erfindungsgemäßen Verbindungen werden dabei in einer Dosis von 10 bis 30 mg/kg p.o. verabreicht. Die Ergebnisse sind in Tabelle 2 angegeben. Bei diesem Test erzielt die bekannte Verbindung Piracetam in einer Dosis von 60 mg/kg p.o. eine prozentuale Abschwächung von 100% (bei 30 mg/kg p.o. 18%).

## Tabelle 2

Prozentuale Abschwächung der scopolamininduzierten Amnesie, erkennbar an einer Verlängerung der Zeit bis zum Betreten des dunklen Teil des Passive-Avoidance Testraumes.

| Verbindungen der allg. Formel I gemäß Beispiel | Dosis mg/kg (p.o.) | Prozentuale Verlängerung |
|---|---|---|
| 1 | 10 | 39 |
| 3 | 10 | 109 |
| 4 | 30 | 142 |
| 6 | 1 i.p. | 282 |
| 13 | 30 | 37 |
| 14 | 30 | 60 |
| 16 | 10 | 114 |
| 17 | 10 | 114 |
| 18 | 10 | 107 |
| 19 | 10 | 30 |
| 23 | 10 | 125 |
| vgl. Piracetam | 30 | 18 |

**Ansprüche**

1. Pyrrolderivate der allgemeinen Formel I

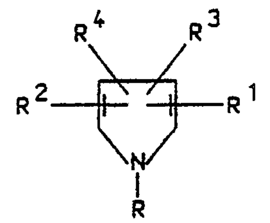

(I)

worin
R Alkyl, das durch $-NH_2$ oder Acylamino der allgemeinen Formel II

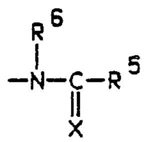

(II)

substituiert ist,
$R^1$, $R^2$ unabhängig voneinander Wasserstoff oder Alkyl mit 1 bis 4 Kohlenstoffatomen,
$R^3$ Wasserstoff, Alkyl mit 1 bis 4 Kohlenstoffatomen oder eine Carbonsäuregruppierung,
$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyc lus mit 1 oder 2 Heteroatomen aus der Reihe N, O, S substituiert ist,
$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5 Kohlenstoffatomen oder gegebenenfalls substituiertes Phenylamino,
$R^6$ Wasserstoff oder Alkyl mit 1 bis 4 Kohlenstoffatomen, wobei $R^5$ und $R^6$ auch zusammen mit der

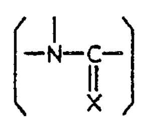 - Gruppe

einen Ring bilden können,
X Sauerstoff oder Schwefel
bedeuten, sowie deren herstellbare, pharmazeutisch akzeptablen Salze bzw. Säureadditionssalze.

2. Pyrrolderivate gemäß Anspruch 1, dadurch gekennzeichnet, daß die für $R^1$, $R^2$ oder $R^3$ stehenden Alkylreste Methyl sind.

3. Pyrrolderivate gemäß den Ansprüchen 1 und/oder 2, dadurch gekennzeichnet, daß die für $R^3$ oder $R^4$ stehenden Carbonsäuregruppierungen Amide, wie zum Beispiel das unsubstituierte Amid, Mono- oder Dialkylamide mit 1 bis 3 Kohlenstoffatomen pro Alkylrest, Ester, wie zum Beispiel Alkylester mit 1 bis 4 Kohlenstoffatomen pro Alkylrest oder Benzylester, die Nitrilgruppe oder die Carboxygruppe selbst bedeuten.

4. Pyrrolderivate nach einem oder mehreren der Ansprüche 1 bis 3, dadurch gekennzeichnet, daß sich die gegebenenfalls substituierten aliphatischen oder aromatischen 5-bis 7-gliedrigen Heterocyclen ableiten von Pyrrol, Pyrrolidin, Imidazol, Thiazol, Thiazolidin oder Perhydrothiazepin.

5. Pyrrolderivate nach einem oder mehreren der Ansprüche 1 bis 4, dadurch gekennzeichnet, daß ein für $R^4$ stehender Phenylsulfinyl-, Phenylsulfonyl-, Phenylcarbonyl- oder ein für $R^5$ stehender Phenyl- oder Phenylamino-Rest sowie ein als Substituent an einen für $R^5$ stehenden Alkylrest gebundener Phenoxyrest im Kern seinerseits bis zu drei Substituenten tragen kann und zwar eine Aminogruppe, Monoalkylamino mit 1 bis 4, vorzugsweise 1 oder 2 Kohlenstoffatomen, Dialkylamino mit insgesamt 2 bis 6, vorzugsweise 2 bis

4, Kohlenstoffatomen, Alkanoylamino mit 1 bis 6, vorzugsweise 1 oder 2, Kohlenstoffatomen, Alkyl mit 1 bis 4, vorzugsweise 1 oder 2, Kohlenstoffatomen, Alkoxy mit 1 bis 4, vorzugsweise 1 oder 2 Kohlenstoffatomen, Halogen, vorzugsweise Fluor, Chlor oder Brom, insbesondere Fluor oder Chlor, Hydroxy, Nitro, Cyano, Carboxy oder Alkoxycarbonyl mit 1 bis 4, vorzugsweise 1 oder 2, Kohlenstoffatomen in der Alkoxygruppe, als gegebenenfalls zweiten Substituenten des Kerns eine der oben definierten Alkyl- oder Alkoxygruppen oder eines der oben genannten Halogene und als gegebenenfalls dritten Substituenten eine der obigen Alkyl- oder Alkoxygruppen.

6. Verfahren zur Herstellung von Pyrrolderivaten der allgemeinen Formel I

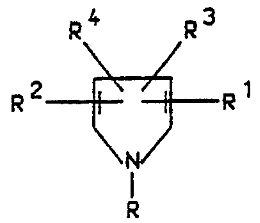

(I)

worin R, $R^1$, $R^2$, $R^3$ und $R^4$ die in den Ansprüchen 1 bis 5 angegebenen Bedeutungen haben, bzw. ihrer pharmazeutisch akzeptablen Salze bzw. Säureadditionssalze, dadurch gekennzeichnet, daß man Pyrrole der allgemeinen Formel III

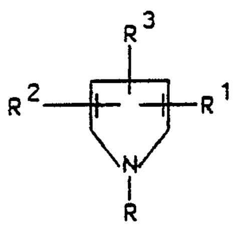

(III)

durch elektrophile Substitution mit Reagenzien, die Reste $R^4$ in den Ring einführen, umsetzt.

7. Verfahren zur Herstellung von Pyrrolderivaten der allgemeinen Formel I

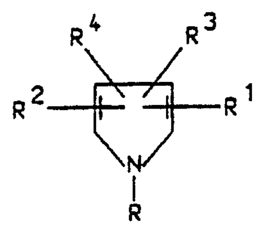

(I)

worin R, $R^1$, $R^2$, $R^3$ die in den Ansprüchen 1 bis 5 angegebenen Bedeutungen haben, und $R^4$ Dicyanmethylen, Alkoxycarbonylvinyl oder Nitril bedeutet, bzw. ihrer pharmazeutisch akzeptablen Salze bzw. Säureadditionssalze, dadurch gekennzeichnet, daß man die entsprechenden Aldehydderivate mit Malonsäuredinitril bzw. Malonsäureestern oder in eine Wittigreaktion bzw. mit Hydroxylamin mit anschließender Wasserabspaltung umsetzt.

8. Verfahren zur Herstellung von Pyrrolderivaten der allgemeinen Formel I

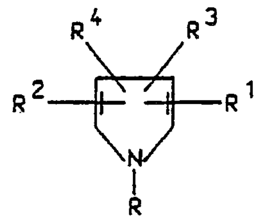

(I)

worin R, $R^1$, $R^2$, $R^3$ und $R^4$ die in den Ansprüchen 1 bis 5 angegebenen Bedeutungen haben, wobei $R^3$ und/oder $R^4$ für eine Carbonsäuregruppierung stehen, bzw. ihrer pharmazeutisch akzeptablen Salze bzw. Säureadditionssalze, dadurch gekennzeichnet, daß man aliphatische 1,4-Dicarbonylverbindungen der allgemeinen Formel V

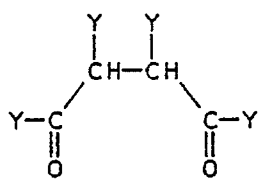

(V)

worin für Y die Reste $R^1$, $R^2$, $R^3$ und $R^4$ je nach gewünschtem Endprodukt stehen, mit Aminen der allgemeinen Formel IV

$H_2N\text{-}R$ (IV)

umsetzt.

9. Verwendung eines Pyrrolderivats der allgemeinen Formel I

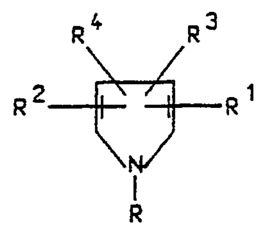

(I)

worin

R Alkyl, das durch $-NH_2$ oder Acylamino der allgemeinen Formel II

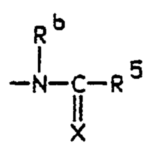

(II)

substituiert ist,

$R^1$, $R^2$ unabhängig voneinander Wasserstoff oder Alkyl mit 1 bis 4 Kohlenstoffatomen,

$R^3$ Wasserstoff, Alkyl mit 1 bis 4 Kohlenstoffatomen oder eine Carbonsäuregruppierung,

$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyclus mit 1 oder 2 Heteroatomen aus der Reihe N, O, S substituiert ist,

$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5

Kohlenstoffatomen oder gegebenenfalls substituiertes Phenylamino,
$R^6$ Wasserstoff oder Alkyl mit 1 bis 4 Kohlenstoffatomen, wobei $R^5$ und $R^6$ auch zusammen mit der

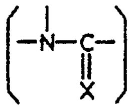 - Gruppe

einen Ring bilden können,
X Sauerstoff oder Schwefel
bedeuten, oder eines herstellbaren, pharmazeutisch akzeptablen Salzes bzw. Säureadditionssalzes davon, zur Herstellung eines Arzneimittels zur Bekämpfung und Vorbeugung von Erkrankungen, die durch Einschränkung der Gehirnfunktion bedingt sind, oder zur Behandlung von cerebralen Alterungsvorgängen.

10. Pharmazeutisches Präparat, dadurch gekennzeichnet, daß es als Wirkstoff ein Pyrrolderivat der allgemeinen Formel I

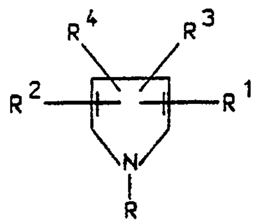

(I)

worin
R Alkyl, das durch $-NH_2$ oder Acylamino der allgemeinen Formel II

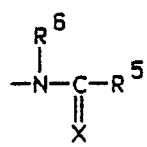

(II)

substituiert ist,
$R^1$, $R^2$ unabhängig voneinander Wasserstoff oder Alkyl mit 1 bis 4 Kohlenstoffatomen,
$R^3$ Wasserstoff, Alkyl mit 1 bis 4 Kohlenstoffatomen oder eine Carbonsäuregruppierung,
$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyclus mit 1 oder 2 Heteroatomen aus der Reihe N, O, S substituiert ist,
$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5 Kohlenstoffatomen oder gegebenenfalls substituiertes Phenylamino,
$R^6$ Wasserstoff oder Alkyl mit 1 bis 4 Kohlenstoffatomen, wobei $R^5$ und $R^6$ auch zusammen mit der

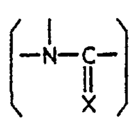 - Gruppe

einen Ring bilden können,

X Sauerstoff oder Schwefel

bedeuten, oder ein herstellbares, pharmazeutisch akzep tables Salz bzw. Säureadditionssalz davon, zusammen mit einem pharmazeutisch annehmbaren Trägerstoff und gegebenenfalls pharmazeutisch annehmbaren Zusatzstoffen und gegebenenfalls noch einen oder mehrere andere pharmakologische Wirkstoffe enthält.

Patentansprüche für folgenden Vertragsstaat: ES

1. Verfahren zur Herstellung von Pyrrolderivaten der allgemeinen Formel I

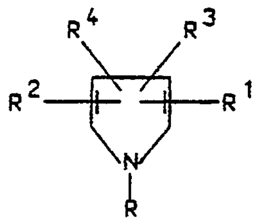

(I)

worin

R Alkyl, das durch $-NH_2$ oder Acylamino der allgemeinen Formel II

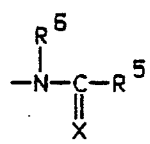

(II)

substituiert ist,

$R^1$, $R^2$ unabhängig voneinander Wasserstoff oder Alkyl mit 1 bis 4 Kohlenstoffatomen,

$R^3$ Wasserstoff, Alkyl mit 1 bis 4 Kohlenstoffatomen oder eine Carbonsäuregruppierung,

$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyclus mit 1 oder 2 Heteroatomen aus der Reihe N, O, S substituiert ist,

$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5 Kohlenstoffatomen oder gegebenenfalls substituiertes Phenylamino,

$R^6$ Wasserstoff oder Alkyl mit 1 bis 4 Kohlenstoffatomen, wobei $R^5$ und $R^6$ auch zusammen mit der

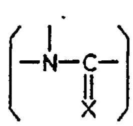 - Gruppe

einen Ring bilden können,

X Sauerstoff oder Schwefel

bedeuten, sowie deren herstellbare, pharmazeutisch akzeptablen Salze bzw. Säureadditionssalze, dadurch gekennzeichnet, daß man Pyrrole der allgemeinen Formel III

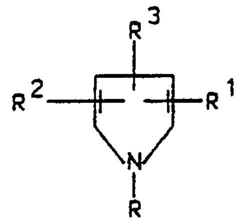

(III)

durch elektrophile Substitution mit Reagenzien, die Reste $R^4$ in den Ring einführen, umsetzt.

2. Verfahren zur Herstellung von Pyrrolderivaten der allgemeinen Formel I

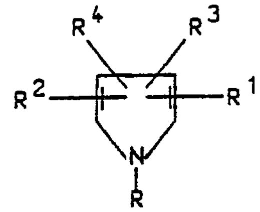

(I)

worin R, $R^1$, $R^2$, $R^3$ die in Anspruch 1 angegebenen Bedeutungen haben, und $R^4$ Dicyanmethylen, Alkoxycarbonylvinyl oder Nitril bedeutet, bzw. ihrer pharmazeutisch akzeptablen Salze bzw. Säureadditionssalze, dadurch gekennzeichnet, daß man die entsprechenden Aldehydderivate mit Malonsäuredinitril bzw. Malonsäureestern oder in eine Wittigreaktion bzw. mit Hydroxylamin mit anschließender Wasserabspaltung umsetzt.

3. Verfahren zur Herstellung von Pyrrolderivaten der allgemeinen Formel I

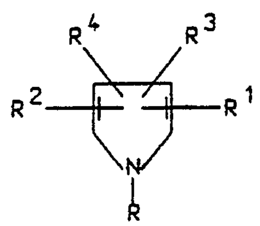

(I)

worin R, $R^1$, $R^2$, $R^3$ und $R^4$ die in den Ansprüchen 1 und/oder 2 angegebenen Bedeutungen haben, wobei $R^3$ und/oder $R^4$ für eine Carbonsäuregruppierung stehen, bzw. ihrer -pharmazeutisch akzeptablen Salze bzw. Säureadditionssalze, dadurch gekennzeichnet, daß man aliphatische 1,4-Dicarbonylverbindungen der allgemeinen Formel V

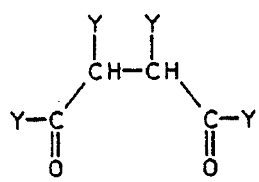

(V)

worin für Y die Reste $R^1$, $R^2$, $R^3$ und $R^4$ je nach gewünschtem Endprodukt stehen, mit Aminen der allgemeinen Formel IV

$H_2N$-R (IV)

umsetzt.

4. Verfahren gemäß einem oder mehreren der Ansprüche 1 bis 3, dadurch gekennzeichnet, daß die für $R^1$, $R^2$ oder $R^3$ stehenden Alkylreste Methyl sind.

5. Verfahren gemäß einem oder mehreren der Ansprüchen 1 bis 4, dadurch gekennzeichnet, daß die für $R^3$ oder $R^4$ stehenden Carbonsäuregruppierungen Amide, wie zum Beispiel das unsubstituierte Amid, Mono- oder Dialkylamide mit 1 bis 3 Kohlenstoffatomen pro Alkylrest, Ester, wie zum Beispiel Alkylester mit

1 bis 4 Kohlenstoffatomen pro Alkylrest oder Benzylester, die Nitrilgruppe oder die Carboxygruppe selbst bedeuten.

6. Pyrrolderivate nach einem oder mehreren der Ansprüche 1 bis 5, dadurch gekennzeichnet, daß sich die gegebenenfalls substituierten aliphatischen oder aromatischen 5-bis 7-gliedrigen Heterocyclen ableiten von Pyrrol, Pyrrolidin, Imidazol, Thiazol, Thiazolidin oder Perhydrothiazepin.

7. Pyrrolderivate nach einem oder mehreren der Ansprüche 1 bis 6, dadurch gekennzeichnet, daß ein für $R^4$ stehender Phenylsulfinyl-, Phenylsulfonyl-, Phenylcarbonyl- oder ein für $R^5$ stehender Phenyl- oder Phenylamino-Rest sowie ein als Substituent an einen für $R^5$ stehenden Alkylrest gebundener Phenoxyrest im Kern seinerseits bis zu drei Substituenten tragen kann und zwar eine Aminogruppe, Monoalkylamino mit 1 bis 4, vorzugsweise 1 oder 2 Kohlenstoffatomen, Dialkylamino mit insgesamt 2 bis 6, vorzugsweise 2 bis 4, Kohlenstoffatomen, Alkanoylamino mit 1 bis 6, vorzugsweise 1 oder 2, Kohlenstoffatomen, Alkyl mit 1 bis 4, vorzugsweise 1 oder 2, Kohlenstoffatomen, Alkoxy mit 1 bis 4, vorzugsweise 1 oder 2 Kohlenstoffatomen, Halogen, vorzugsweise Fluor, Chlor oder Brom, insbesondere Fluor oder Chlor, Hydroxy, Nitro, Cyano, Carboxy oder Alkoxycarbonyl mit 1 bis 4, vorzugsweise 1 oder 2, Kohlenstoffatomen in der Alkoxygruppe, als gegebenenfalls zweiten Substituenten des Kerns eine der oben definierten Alkyl- oder Alkoxygruppen oder eines der oben genannten Halogene und als gegebenenfalls dritten Substituenten eine der obigen Alkyl- oder Alkoxygruppen.

8. Verwendung eines Pyrrolderivats der allgemeinen Formel I

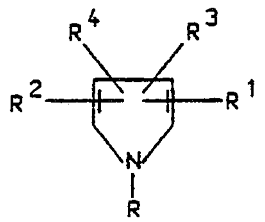

(I)

worin

R Alkyl, das durch $-NH_2$ oder Acylamino der allgemeinen Formel II

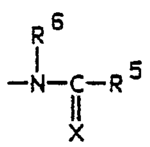

(II)

substituiert ist,

$R^1$, $R^2$ unabhängig voneinander Wasserstoff oder Alkyl mit 1 bis 4 Kohlenstoffatomen,

$R^3$ Wasserstoff, Alkyl mit 1 bis 4 Kohlenstoffatomen oder eine Carbonsäuregruppierung,

$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyclus mit 1 oder 2 Heteroatomen aus der Reihe N, O, S substituiert ist,

$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5 Kohlenstoffatomen oder gegebenenfalls substituiertes Phenylamino,

$R^6$ Wasserstoff oder Alkyl mit 1 bis 4 Kohlenstoffatomen, wobei $R^5$ und $R^6$ auch zusammen mit der

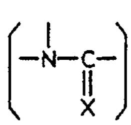 - Gruppe

einen Ring bilden können,
X Sauerstoff oder Schwefel
bedeuten, oder eines herstellbaren, pharmazeutisch akzeptablen Salzes bzw. Säureadditionssalzes davon, zur Herstellung eines Arzneimittels zur Bekämpfung und Vorbeugung von Erkrankungen, die durch Einschränkung der Gehirnfunktion bedingt sind, oder zur Behandlung von cerebralen Alterungsvorgängen.

9. Verfahren zur Herstellung eines pharmazeutischen Präparates, dadurch gekennzeichnet, daß ein oder mehrere Pyrrolderivate der allgemeinen Formel I

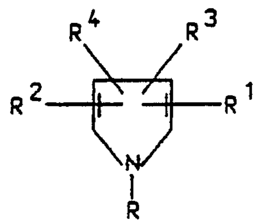

(I) .

worin
R Alkyl, das durch $-NH_2$ oder Acylamino der allgemeinen Formel II

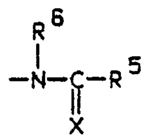

(II)

substituiert ist,
$R^1$, $R^2$ unabhängig voneinander Wasserstoff oder Alkyl mit 1 bis 4 Kohlenstoffatomen,
$R^3$ Wasserstoff, Alkyl mit 1 bis 4 Kohlenstoffatomen oder eine Carbonsäuregruppierung,
$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyclus mit 1 oder 2 Heteroatomen aus der Reihe N, O, S substituiert ist,
$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5 Kohlenstoffatomen oder gegebenenfalls substituiertes Phenylamino,
$R^6$ Wasserstoff oder Alkyl mit 1 bis 4 Kohlenstoffatomen, wobei $R^5$ und $R^6$ auch zusammen mit der

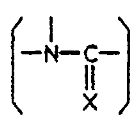 - Gruppe

einen Ring bilden können,
X Sauerstoff oder Schwefel
bedeuten, oder ein herstellbares, pharmazeutisch akzeptables Salz bzw. Säureadditionssalz davon durch Mischen mit einem pharmazeutisch annehmbaren Trägerstoff und gegebenenfalls pharmazeutisch annehmbaren Zusatzstoffen und gegebenenfalls mit einem oder mehreren anderen pharmakologischen Wirkstoffen in eine zur Verabreichung geeignete Form gebracht werden.

Patentansprüche für folgenden Vertragsstaat: GR

1. Pyrrolderivate der allgemeinen Formel I

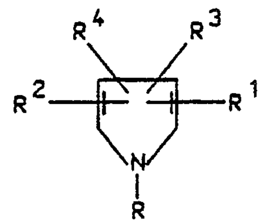

(I)

worin
R Alkyl, das durch $-NH_2$ oder Acylamino der allgemeinen Formel II

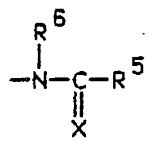

(II)

substituiert ist,
1 bis 4 Kohlenstoffatomen,
$R^3$ Wasserstoff, Alkyl mit 1 bis 4 Kohlenstoffatomen oder eine Carbonsäuregruppierung,
$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyc lus mit 1 oder 2 Heteroatomen aus der Reihe N, O, S substituiert ist,
$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5 Kohlenstoffatomen oder gegebenenfalls substituiertes Phenylamino,
wobei $R^5$ und $R^6$ auch zusammen mit der

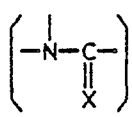 - Gruppe

einen Ring bilden können,
X Sauerstoff oder Schwefel
bedeuten, sowie deren herstellbare, pharmazeutisch akzeptablen Salze bzw. Säureadditionssalze.

2. Pyrrolderivate gemäß Anspruch 1, dadurch gekennzeichnet, daß die für $R^1$, $R^2$ oder $R^3$ stehenden Alkylreste Methyl sind.

3. Pyrrolderivate gemäß den Ansprüchen 1 und/oder 2, dadurch gekennzeichnet, daß die für $R^3$ oder $R^4$ stehenden Carbonsäuregruppierungen Amide, wie zum Beispiel das unsubstituierte Amid, Mono- oder Dialkylamide mit 1 bis 3 Kohlenstoffatomen pro Alkylrest, Ester, wie zum Beispiel Alkylester mit 1 bis 4 Kohlenstoffatomen pro Alkylrest oder Benzylester, die Nitrilgruppe oder die Carboxygruppe selbst bedeuten.

4. Pyrrolderivate nach einem oder mehreren der Ansprüche 1 bis 3, dadurch gekennzeichnet, daß sich die gegebenenfalls substituierten aliphatischen oder aromatischen 5-bis 7-gliedrigen Heterocyclen ableiten von Pyrrol, Pyrrolidin, Imidazol, Thiazol, Thiazolidin oder Perhydrothiazepin.

5. Pyrrolderivate nach einem oder mehreren der Ansprüche 1 bis 4, dadurch gekennzeichnet, daß ein für $R^4$ stehender Phenylsulfinyl-, Phenylsulfonyl-, Phenylcarbonyl- oder ein für $R^5$ stehender Phenyl- oder Phenylamino-Rest sowie ein als Substituent an einen für $R^5$ stehenden Alkylrest gebundener Phenoxyrest im Kern seinerseits bis zu drei Substituenten tragen kann und zwar eine Aminogruppe, Monoalkylamino mit 1 bis 4, vorzugsweise 1 oder 2 Kohlenstoffatomen, Dialkylamino mit insgesamt 2 bis 6, vorzugsweise 2 bis

4, Kohlenstoffatomen, Alkanoylamino mit 1 bis 6, vorzugsweise 1 oder 2, Kohlenstoffatomen, Alkyl mit 1 bis 4, vorzugsweise 1 oder 2, Kohlenstoffatomen, Alkoxy mit 1 bis 4, vorzugsweise 1 oder 2 Kohlenstoffatomen, Halogen, vorzugsweise Fluor, Chlor oder Brom, insbesondere Fluor oder Chlor, Hydroxy, Nitro, Cyano, Carboxy oder Alkoxycarbonyl mit 1 bis 4, vorzugsweise 1 oder 2, Kohlenstoffatomen in der Alkoxygruppe, als gegebenenfalls zweiten Substituenten des Kerns eine der oben definierten Alkyl- oder Alkoxygruppen oder eines der oben genannten Halogene und als gegebenenfalls dritten Substituenten eine der obigen Alkyl- oder Alkoxygruppen.

6. Verfahren zur Herstellung von Pyrrolderivaten der allgemeinen Formel I

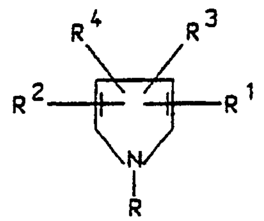

(I)

worin R, $R^1$, $R^2$, $R^3$ und $R^4$ die in den Ansprüchen 1 bis 5 angegebenen Bedeutungen haben, bzw. ihrer pharmazeutisch akzeptablen Salze bzw. Säureadditionssalze, dadurch gekennzeichnet, daß man Pyrrole der allgemeinen Formel III

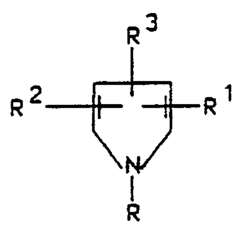

(III)

durch elektrophile Substitution mit Reagenzien, die Reste $R^4$ in den Ring einführen, umsetzt.

7. Verfahren zur Herstellung von Pyrrolderivaten der allgemeinen Formel I

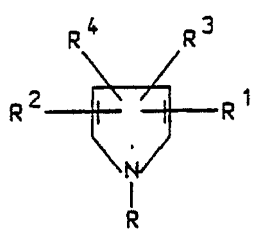

(I)

worin R, $R^1$, $R^2$, $R^3$ die in den Ansprüchen 1 bis 5 angegebenen Bedeutungen haben, und $R^4$ Dicyanmethylen, Alkoxycarbonylvinyl oder Nitril bedeutet, bzw. ihrer pharmazeu tisch akzeptablen Salze bzw. Säureadditionssalze, dadurch gekennzeichnet, daß man die entsprechenden Aldehydderivate mit Malonsäuredinitril bzw. Malonsäureestern oder in eine Wittigreaktion bzw. mit Hydroxylamin mit anschließender Wasserabspaltung umsetzt.

8. Verfahren zur Herstellung von Pyrrolderivaten der allgemeinen Formel I

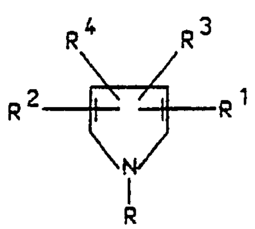

(I)

worin R, $R^1$, $R^2$, $R^3$ und $R^4$ die in den Ansprüchen 1 bis 5 angegebenen Bedeutungen haben, wobei $R^3$ und/oder $R^4$ für eine Carbonsäuregruppierung stehen, bzw. ihrer pharmazeutisch akzeptablen Salze bzw. Säureadditionssalze, dadurch gekennzeichnet, daß man aliphatische 1,4-Dicarbonylverbindungen der allgemeinen Formel V

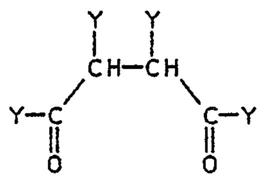

(V)

worin für Y die Reste $R^1$, $R^2$, $R^3$ und $R^4$ je nach gewünschtem Endprodukt stehen, mit Aminen der allgemeinen Formel IV

$H_2N$-R (IV)

umsetzt.

9. Verwendung eines Pyrrolderivats der allgemeinen Formel I

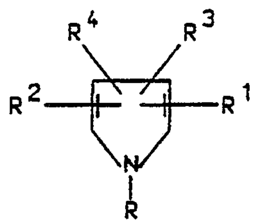

(I)

worin

R Alkyl, das durch $-NH_2$ oder Acylamino der allgemeinen Formel II

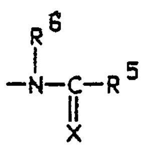

(II)

substituiert ist,

$R^1$, $R^2$ unabhängig voneinander Wasserstoff oder Alkyl mit 1 bis 4 Kohlenstoffatomen,

$R^3$ Wasserstoff, Alkyl mit 1 bis 4 Kohlenstoffatomen oder eine Carbonsäuregruppierung,

$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyclus mit 1 oder 2 Heteroatomen aus der Reihe N, O, S substituiert ist,

$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5 Kohlenstoffatomen oder gegebenenfalls substituiertes Phenylamino,

$R^6$ Wasserstoff oder Alkyl mit 1 bis 4 Kohlenstoffatomen, wobei $R^5$ und $R^6$ auch zusammen mit der

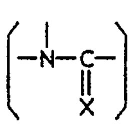 - Gruppe

einen Ring bilden können,
X Sauerstoff oder Schwefel
bedeuten, oder eines herstellbaren, pharmazeutisch akzeptablen Salzes bzw. Säureadditionssalzes davon, zur Herstellung eines Arzneimittels zur Bekämpfung und Vorbeugung von Erkrankungen, die durch Einschränkung der Gehirnfunktion bedingt sind, oder zur Behandlung von cerebralen Alterungsvorgängen.

10. Verfahren zur Herstellung eines pharmazeutischen Präparates, dadurch gekennzeichnet, daß ein oder mehrere Pyrrolderivate der allgemeinen Formel I

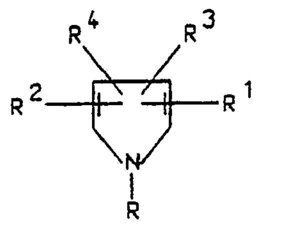

(I)

worin
R Alkyl, das durch $-NH_2$ oder Acylamino der allgemeinen Formel II

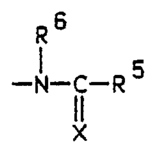

(II)

substituiert ist,
$R^1$, $R^2$ unabhängig voneinander Wasserstoff oder Alkyl mit 1 bis 4 Kohlenstoffatomen,
$R^3$ Wasserstoff, Alkyl mit 1 bis 4 Kohlenstoffatomen oder eine Carbonsäuregruppierung,
$R^4$ eine Carbonsäuregruppierung, Nitro, Alkylsulfinyl mit 1 bis 4 Kohlenstoffatomen, gegebenenfalls substituiertes Phenylsulfinyl, Alkylsulfonyl mit 1 bis 4 Kohlenstoffatomen, gegebenenfalls substituiertes Phenylsulfonyl, Alkylcarbonyl mit 1 bis 6 Kohlenstoffatomen im Alkylteil, Trifluormethylcarbonyl, gegebenenfalls substituiertes Phenylcarbonyl, Dicyanmethylen, Formylvinyl, Alkoxycarbonylvinyl mit 1 bis 6 Kohlenstoffatomen im Alkoxyteil oder eine Carbonylgruppe, die durch einen gegebenenfalls substituierten aliphatischen oder aromatischen 5- bis 7-gliedrigen Heterocyclus mit 1 oder 2 Heteroatomen aus der Reihe N, O, S substituiert ist,
$R^5$ Wasserstoff, gegebenenfalls substituiertes Alkyl, Cycloalkyl mit 5 bis 7 Kohlenstoffatomen, gegebenenfalls substituiertes Phenyl, einen gegebenenfalls substituierten aliphatischen oder aromatischen 5- bis 7-gliedrigen Heterocyclus mit 1 oder 2 Heteroatomen aus der Reihe N, O, S, Amino, Alkylamino mit 1 bis 5 Kohlenstoffatomen oder gegebenenfalls substituiertes Phenylamino,
$R^6$ Wasserstoff oder Alkyl mit 1 bis 4 Kohlenstoffatomen, wobei $R^5$ und $R^6$ auch zusammen mit der

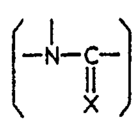 - Gruppe

einen Ring bilden können,
X Sauerstoff oder Schwefel
bedeuten, oder ein herstellbares, pharmazeutisch akzeptables Salz bzw. Säureadditionssalz davon, durch Mischen mit einem pharmazeutisch annehmbaren Trägerstoff und gegebenenfalls pharmazeutisch annehmbaren Zusatzstoffen und gegebenenfalls mit einem oder mehreren anderen pharmakologischen Wirkstoffen in eine zur Verabreichung geeignete Form gebracht werden.

Europäisches Patentamt

# EUROPÄISCHER RECHERCHENBERICHT

Nummer der Anmeldung

EP 89109808.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 527 791 (CASSELLA AG) * Zusammenfassung; Seite 7, Zeilen 9-13 * ---- | 1,8-10 | C 07 D 207/327 |

| RECHERCHIERTE SACHGEBIETE (Int. Cl.4) |
|---|
| C 07 D 207/00 |

Der vorliegende Recherchenbericht wurde für alle Patentansprüche erstellt.

| Recherchenort | Abschlußdatum der Recherche | Prüfer |
|---|---|---|
| WIEN | 07-09-1989 | HEIN |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument